# EUROPEAN PATENT APPLICATION

(11) **EP 0 908 521 A1**
(43) Date of publication of application: **14.04.1999**
(21) Application number: 97117574.0
(22) Date of filing: 10.10.1997
(51) Int. Cl.: C12N 15/85, A61K 48/00

(54) **Transfection system for the transfer of nucleic acids into cells**

(71) Applicant: Hoechst Marion Roussel Deutschland GmbH, 65929 Frankfurt am Main (DE)
(72) Inventor: Chandra, Prakash, Prof.Dr., 60489 Frankfurt (DE); Chandra, Angelika, Dr., 60489 Frankfurt (DE); Dermirhan, Ilhan, Dr., 65931 Frankfurt (DE); Hasselmayer, Oliver, Dr., 63065 Offenbach (DE)

(57) **Abstract**

This invention relates to a transfection system comprising histones or derivates thereof and nucleic acids that should be brought into cells, the use of the transfection system for any kind of nucleic acid transfection, especially the use ot the transfection system for gene therapy and for the preparation of pharmaceuticals.

## Description

This invention relates to a new transfection system comprising of histones or derivates thereof and nucleic acids that should be brought into cells, the use of the new transfection system for any kind of nucleic acid transfection, especially the use of the new transfection system for gene therapy and for the preparation of pharmaceuticals.

One of the major problems when foreign genes should be introduced and expressed in eukaryotic cells is that the DNA must cross several membranous barriers to reach and enter the cell nucleus. The first barrier, the cytoplasmic membrane, can be traversed by many different methods (for reviews, see Keown et al., 1990) like calcium phosphate precipitation (Loyter et al., 1982; Chen et al., 1988), electroporation (Neumann et al., 1982; Othani et al., 1989), liposome packaging of DNA (Fraley et al., 1982; Nicolau et al., Mannino et al., 1988), with (Wang et al. 1987; Machy et al. 1988; Kanedi et al., 1989) or without specific targeting of the liposomes, lipofection (Felgner et al., 1987; Holt et al., 1990) and microinjection (Graessmann et al., 1986; Wolff et al., 1990; Ascadi et al., 1991). Also described is the use of polycations such as Polybrene (Kawai et al., 1984), DEAE-dextran (Chandra et al., 1988; Demirhan et al., 1994; Graham et al., 1973; McCutchan et al., 1968; Choi et al., 1988) or the use of proteins, which are taken up by the process of receptor-mediated endocytosis (Wagner et al., 1990; Zenke et al., 1990; Wu et al., 1989; Wu et al., 1991). The method of receptor-mediated gene transfer is a specific approach, but it is limited by the presence and the number of receptors, which can be modulated in certain cases (Cotten et al., 1990; Marceau et al., 1990; Curiel et al., 1992).

Furthermore, there are other several drawbacks that limit foreign gene delivery and expression in target cells. Such limitations include insufficient condensation of plasmid DNA and the lack of specific mechanisms for DNA to escape from endosomes, and to enter the cell nucleus. There are described some ways to improve these methods and compensate the above mentioned drawbacks like an increase in reporter gene expression when plasmid DNA is compactly packed into toroid structure by protein carriers (Wagner et al., 1991), which is dependent upon having the right combination of the length and the amount of the DNA-binding part in the carrier complex. To increase the escape of DNA from endosomes, some reports describe to the use of the property of some viruses to destroy endosomes (Curiel et al., 1991; Wagner et al., 1992a; Wagner et al., 1992b; Cotten et al., 1992; Cotten et al., 1993; Plank et al., 1994; Wu et al., 1994; Zauner et al., 1995).

The present invention aims to provide a universal transfection system that can be used to bring nucleic acids into prokaryotic and/or eukaryotic cells, in particular into the nuclei of eukaryotic target cells in a simple and effective manner.

According to the invention the problem is solved by providing a transfection system that comprises 1. at least one basic protein that tend to aggregate with nucleic acids and which have a nuclear localization signal (NLS) responsible for targeting proteins to the nucleus and 2. at least one nucleic acid that should be transferred to the nucleus and 3. optionally additional compounds.

Transfection means that nucleic acids such as for example DNA are brought into eukaryotic cells, in particular transfection means that the nucleic acids are brought into the nucleus of an eukaryotic cell. But the transfection system can also be used to bring nucleic acids into prokaryotic cells.

One of the most favorable embodiments of the invention relates to a transfection system that comprises 1. a histone protein or derivate thereof and 2. a nucleic acid that should be transported and 3. optionally additonal compounds.

Histones are basic proteins which are components of the chromatin structures naturally occuring in most of the nuclei of eukaryotic cells. Histones are characterized by having an unusually high amount of basic amino acids (about 20 to 30 %) and a relative molecular weight of about 11.000 to 24.000 Da (e.g. Benjamin Lewin (1983) "Genes III", John Wiley & Sons, Inc. New York, p. 519-558).

Five different classes of histones are known: The histones 2A, 2B, 3 and 4 (H2A, H2B, H3, H4) form a histone octamer which interacts directly with DNA to form the first level of particles in the chromatin , whereas the histone 1 (H1) is concerned with interactions between these particles.

The histones H3 and H4 are the most Arginine-rich histones. For example bovine histone H3 contains about 13 % Arginin, 10 % Lysin and has a relative molecular weight of about 15,3 Da. The bovine histone H4 contains about 14 % Arginin, 11 % Lysin and has a relative molecular weight of about 11,2 to 11,3 Da.

The histones H3 and H4 are among the most conserved proteins known in evolution. For example the sequences of the 102 amino acid H4 of the cow differs only in two amino acids from that of the pea and the sequences of the 135 amino acid H3 of the cow differs in 4 amino acids from that of the pea (Lexikon der Biochemie und Molekularbiologie (1991) Herder Verlag, Freiburg).

Histone 3 and histone 4 are small positivly charged proteins which can bind nucleic acids by ionic interactions. In addition H3 and H4 have nuclear location signal sequences. This property leads to rapid accumulation of H3 and H4 in the nucleus both by diffusion trought and active transport into the nucleus (Bonner, 1975; Dingwall *et al*., 1984; Moreland et al., 1987).

In a preferred embodiment of the invention, the transfection system can comprises histone 3 (H3) and/or a derivate thereof and/or histone 4 (H4) and/or a derivate thereof. In another preferred embodiment the invention comprises a mixture of histones and optionally other compounds.

Derivates of histones comprise any histones, in particular naturally occuring histones that have been modified or altered in any way with the provise that their specific ability to aggregate with nucleic acids and translocate them into the nuclei of cells is maintained. Modifications are for example deletion and/or addition of one or more amino acids from/to the aminoterminal and/or from/to the carboxyterminal end of the histone protein and/or from/to somewhere in the middle of the amino acid sequence. Further examples for alterations are amino acid changes or amino acid substitutions respectively within the naturally occuring amino acid sequence of histones and/or modifications of amino acids, e.g. acetylation (e.g. ε-aminogroup of lysine residues), ADP-ribolysation (e.g. glutamine residues), methy]ation and/or phosphorylation (e.g. hydroxy groups of serine and/or threonine residues).

The invention also comprises the process of preparation of the transfection system. The transfection system can be produced by incubating histones with nucleic acids. In a special embodiment the incubation time is about 5 to 60 minutes, preferably 10 to 30 minutes, most preferably about 15 minutes.

For transfection or in the transfection system or for the preparation of the transfection system respectively e.g. histone proteins that have been isolated and/or recombinant histones can be used.

The histones can for example be isolated from any kind of cells and/or cell lines, any kind of animal tissue, preferably thymus or from human/animal, umbilical cords, human/animal transplantation waste product, human/animal biopsy-material, human/animal tumor tissue (e.g. material which has been removed by operation surgery) and/or human/animal placenta. The preparation of histones from tissue and/or cells is for example described in E.W. Johns, Biochem. J. 92, 55-59 (1964).

Recombinant histones can be obtained by expression of histone-encoding DNA in prokaryotic and/or eukaryotic cells. For example human histones can be obtained by cloning human H3-cDNA (for example a DNA encoding for the amino acid sequence in table 7) and/or human H4-cDNA (for example a DNA encoding for the amino acid sequence in table 5) in an appropiate (expression-) vector and expression in suitable host cells. Histones can also be obtained from transgenic animals which (over)-express a particular histone-transgene.

Sequences of histone encoding DNA can be obtained from genetic sequence databases such as for example from the NCBI (National Center for Biotechnology Information), EMBL (European Molecular Biology Laboratory) and/or the Internet for example under http://www.ncbi.nlm.nih.gov.Baxevani/HISTONES/table.html.

Some histones are also commercially available, e.g. H3 and H4 (isolated from calf thymus) and can be obtained from Fluka Chemie AG, Neu-Ulm (No. 53411, Histone H3, Johns Fraction f3; No. 53413, Histone H4, Johns Fractione f2a). The histones from Fluka are prepared by a modified preparation process according to E.W. Johns, Biochem. J. 92, 55-59 (1964).

Histones from any kind of animal and/or eukaryotic microorganism and/or plant can be used, e.g. from vertebrates and/or mammalians such as for example rodents, primates, sheep, goat, dog, cow, pig, birds and/or amphibians, reptiles. This is of particular interest, since for example H3 and H4 are highly conserved between different species. The consensus sequence for H3 is given in table 6. The amino acid sequence of human H3 (table7) differs only in 7 positons (5 within the sequence and 2 at the carboxy terminal end) from the consensus sequence. The consensus sequence for H4 is given in table 4. The amino acid sequence of human H4 (table 5) differs only in 3 amino acids from the consensus sequence.

Nucleic acids which can be transferred are for example DNA, RNA, oligonucleotides or derivates thereof. Derivates are for example nucleic acids in which the phosphodiester internucleoside linkage, the sugar unit, the nucleobase and/or the sugar-phosphate backbone has been modified and/or replaced. Derivates include also nucleic acid salts, in particular physiological tolerated salts thereof.

The nucleic acids can for example be isolated and/or synthesized by chemical and/or enzymatical methodes. The nucleic acid may have any kind of length. For example nucleic acids may be relatively short, for example comprise only 10 or less, 20, 30, 40, 50 or 100 nucleotides. On the other hand nucleic acids may comprise 1000, 10000, 100000, 1000000 or more nucleotides. The nucleic acid may be a chromosome.

DNA comprises for example any kind of gene, derivates and/or parts thereof, any kind of cDNA, derivates and/or parts thereof. The DNA can be linear and/or circular (e.g. plasmid, cosmid, artificial chromosome).

The nucleic acid may encode for one or more proteins, in particular soluble and/or unsoluble and/or membrane bound proteins. Nucleic acids may encode for example for peptides, enzymes, receptors, cell-adhesion proteins, immunomodulators, growth factors, tumorsupressors, regulatory proteins such as transcriptionfactors, co-factors and/or trans-activating factors.

Nucleic acids may function, in particular if the nucleic acid is RNA and/or oligonucleotide as antisense oligonucleotide, as triplex-forming oligonucleotide and/or as ribozyme (e.g hammerhead-ribozyme).

The nucleic acid source may be any human and or non-human animal, plant, microorganism such as e.g. bacteria, fungi (e.g. yeast), virus, retrovirus.

In a special embodiment of the invention the DNA encodes among others for the enzyme Chloramphenicol-Acetyl-Transferase (CAT).

In another special embodiment of the invention the DNA encodes beyond others for the HIV-1 protein tat.

Further, non-limiting examples of genes/cDNAs/coding sequences which could be (part of) the nucleic acid are immun-modulating genes such as those of cytokines like GM-CSF, IFN-γ, IL-2, alloantigen genes such as HLA-B7, "suicide" genes such as HSV-thymidine kinase, co-stimmulatory genes such as B7-1, tumor-associated antigenes, such as MART-1, tumor-suppressor genes such as p53, antisense genes that target oncogenes, antigrowth-factor genes, drug resistance genes.

The nucleic acid may comprise the sequences of one or more gene-regulatory sequences for controlled expression, e.g. natural and/or synthetic promotors and/or enhancers. Promotors may be constiutive and/or inducible. The nucleic acid can comprise basal promotor, viral promotor, promotors which are activated upon metabolic stimulation or upon treatment with a specific drug (e.g. tetracyclin), cell-cycle specific promotors, cell-type specific promotors and/or chimeric promotors.

In a special embodiment of the invention the nucleic acid comprises contains the adenovirus major late promoter.

In another embodiment of the invention the nucleic acid comprises an inducable promoter which is acitvated upon stimulation with a specific trans-activating factor. In another special embodiment the inducible promotor is the HIV-1 long terminal repeat (LTR). This promotor is stimulated by the trans-activating factor tat which is specific for a particular cell type since its expression is restricted to HIV-infected cells.

In a special embodiment of the invention the transfection system may comprise different ratios of nucleic acid and histon. In special embodiments the transfection system may comprise a nucleic acid to histone ratio of for example about 1 to 5, 1 to 4,5, 1 to 4, 1 to 3,5, 1 to 3, 1 to 2,5, 1 to 2, 1 to 1,5, 1 to 1 or less. In other special embodiments the transfection system may comprise a nucleic acid to histone ratio of about 1 to 5,5, 1 to 6, 1 to 6,5 1 to 7, 1 to 8, 1 to 9, 1 to 10, 1 to 100 or more. In other special embodiments of the invention the nucleic acid to histone ratio may be 1,5 to 1, 2 to 1, 2,5 to 1, 3 to 1, 3,5 to 1, 4 to 1, 5 to 1, 10 to 1, 100 to 1 or more.

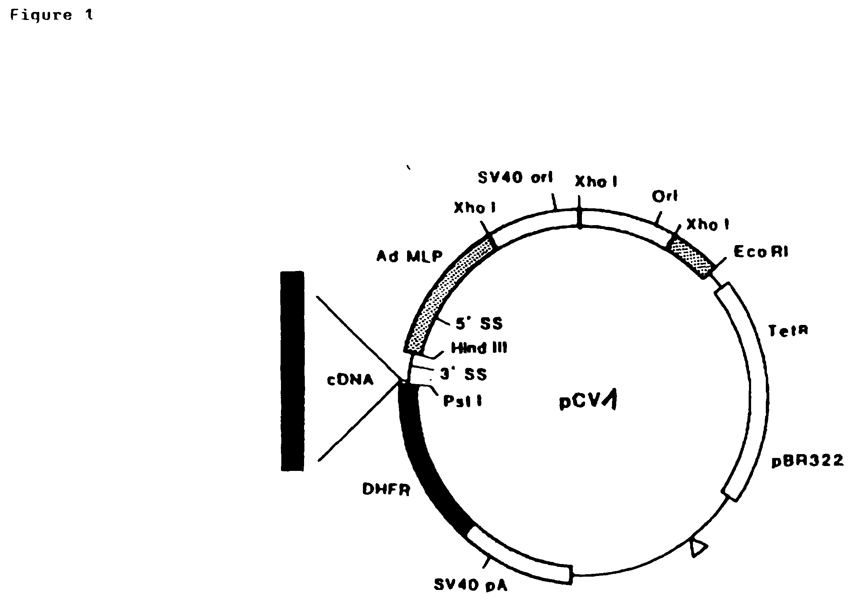

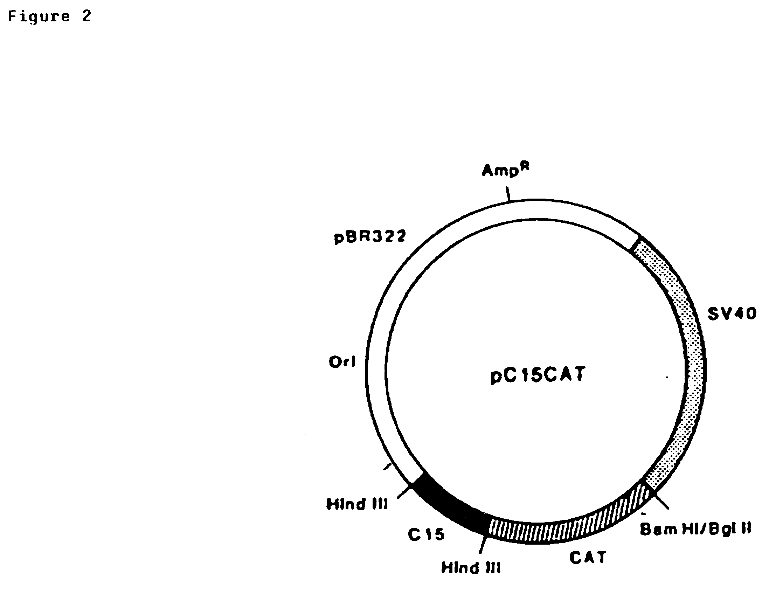

In a special embodiment of the invention the nucleic acid comprise one or more plasmids. These are for example the plasmids pCV1 (comprises tat gene and SV40 promotor) and pC15CAT (comprises HIV-1 LTR and CAT). The plasmids pCV1 (figure 1) and pC15CAT (figure 2) are described in Chandra et al. (1988) FEBS letters, Vol. 236, 282-286.

The transfection system may comprise additional compounds. These compounds are for example proteins, peptides, amino acids, chemical substances. Histones prepared from tissue and/or cells and/or cell nuclei may for example comprise additional compounds such as for example cofactors. If histones that have been isolated from tissue were used in the transfection system, the transfection system may comprise additional compounds assocciated with histones and/or contained in the histone fraction such as for example in Johns fraction f3 and/or 22a.

The new transfection system can be universal applied, e.g. whenever nucleic acids should be brought into (the nuclei of) target cells or whenever genetically engineered eukaryotic cells should be provided.

Target cells are any types of eukaryotic cells and/or cell lines, cells of tissues and/or tissue cultures and/or cells of animals and/or plants and or prokaryotic cells. Non-limiting examples are endothel cells, muscle cells, hamopoetic cells, steam cells, cells belonging to the immuno system (e.g. T-cells, B-cells, lymphoctes, macrophages), glia cells, tumor cells and/or derivates thereof.

In a special embodiment of the invention the transfection system is used to bring nucleic acids into the nuclei of Jurkat cells.

In special embodiments of the invention the transfection time (incubation time of transfection system with cell(s)) may be about 10 minutes (min) or less, 30 min, 45 min, 60 min, 1,5 hours (hrs),2 hrs, 2,5 hrs, 3 hrs, 4 hrs, 5 hrs, 6 hrs, 7 hrs, 8 hrs, 9 hrs, 10 hrs, 11 hrs, 12 hrs, 14 hrs, 16 hrs, 18 hrs, 20 hrs, 24 hrs, 1,5 days (d), 2 d, 2,5 d, 3 d, 3,5 d, 4 d, 4,5 d, 5 d, 6 d, 7 d or longer.

The new transfection system can for example be used in diagnosis and for the preparation of diagnostic tests. It can furthermore be used to prepare cells for the use in screening assays, e.g. for the screening of new protein and/or nucleic acid targets and/or the screening of highly specific new drugs.

The new transfection system can be used for DNA-vaccination and/or for the preparation of a pharmaceutical composition that can be used as DNA-vaccines and/or as immune therapeutics.

The new transfection system can be used in gene therapy, in particular for somatic gene therapy and/or for the preparation of pharmaceutical compositions that can be used for gene therapy, in particular for somatic gene therapy. The pharmaceutical composition should comprise an effective amount of the transfection system. The transfection system can be used for ex vivo and/or in vivo application.

For ex vivo application for example hemapoetic stem cells (e.g. from born marrow) can be transfected with the transfection system. Depending on the nucleic acid that is used transfected stem cells may for example be enabled to target specific cells, e.g. tumor cells and optionally deliver e.g. nucleic acids to those target cells.

For in vivo application for example the use of a specific nucleic acid, e.g. with a promotor which is acivated only in specific cell type(s) and/or at a specific point of development and/or differentiation and/or cell cycle can ensure ashured that the encoded protein is expressed in particular target cells, optionally at a special point of development/differentiation/cell cycle. For in vivo application the transfection system and/or a pharmaceutical composition thereof can for example be injected into particular tissue. This is possible because the transfection system is taken up by cells very efficiently. This methode may therefore be used for in vivo gene therapy of solid tumors.

The transfection system or a pharmaceutical composition thereof may be an aerosol that can be inhaled, it may in a liquid that can be injected or drunken or it may be in a solid form for oral uptake.

The transfection system or a pharmaceutical composition thereof can be used for the diagnosis, the prevention or the treatment of diseases, for example different kinds of cancer such as for example melanoma, carcinoma, lymphoma, basalioma, sarkoma, adenoma, neuroblastoma, glioma, tetraoma, haemoblastoma in different kinds of tissues like for example head, brain, neck, skin, breast, stomache, kidney, bone, hepatic system, muscle, lung, prostate and different kinds of infections such as for example infections with different kinds of Influenza-virus, HIV (human immunodeficiency virus), HSV (herpes simplex virus), CMV (cytomegalo virus), HBV (hepatitis B-virus), HCV (hepatitis C-virus) and other types of hepatitis viruses, heliobacter plyori, malaria, cholera, thyphus, grain and different kinds of genetic diseases such as for example cystric fibrosis, thalassaemia, cardiovascular diseases like atheriosclerosis, retenosis and different kinds of immunological diseases such as for example autoimmuno diseases and different kinds of degenerative diseases such as for example Alzheimer's diseases, osteoporosis and diabetes, asthma, obesity.

### Detailed description of the invention

Some lipofection reagents have been studied in comparison to DEAE-dextran gene delivery system. DOTAP, Dosper and lipofectin have shown lower transfection efficiency. However, lipofectamine had a 2,5-fold better efficiency compared to that of DEAE-dextran. This could be due to it's stronger cationic character leading to a better condensation of DNA. This invention provides a novel and highly efficient DNA carrier system using the natural DNA-binding proteins, histones 3 and 4. Both proteins have nuclear location signal sequences which are responsible for their rapid accumulation in the cell nucleus. This transfection method (histonefection) is simple and not targeted to specific cell surface receptors. In transfection experiments the transactivation of HIV-1 LTR by the transactivator protein tat, both expressed in Jurkat cells was determined. HIV-1 LTR was hybridized to the gene of CAT which was used as reporter gene. In the experiments the level of expression of CAT was a direct measure of transactivation.

Compared to DEAE-dextran mediated transfection, histonefection resulted in a 7-fold increase of CAT expression. The maximum transfection efficiency of histones is dependent on concentration (DNA : histone ratio) and the incubation period. In a gel-retardation assay (gel shift assay) a maximum complex formation (between histone and DNA) was observed under the same conditions needed for the highest transfection efficiency. This ability of histones to increase the delivery and transgenic expression of foreign DNA in eukaryotic cells is not due to the positive ionic character of histone proteins since in experiments of the invention poly-lysine failed to catalyze transfection. This invention provides a simple histone-mediated gene delivery system that can be used to translocate foreign genes into target cells.

The nuclear location and natural DNA-binding capability of histones make them ideal for delivering DNA into the cell nuleus. The simple construction of the histone-based carrier system means less variables need to be controlled, and there is a greater reliability in this method as compared with systems, which used receptor-mediated endocytose. In order to construct a natural ligandpolylysine complex multiple steps of modification, purification and components were required (Wagner et al., 1991). On the one hand the renunciation of natural ligand to cell surface receptors by the use of unmodified histones in this method is potentially applicable to eukaryotic cells without specific surface receptors. On the other hand the transfection with histones is more efficient than all the other tested transfection reagents. In comparison to transfection efficiency of DEAE-dextrane the histones mediates a 7-fold increase in transfection efficiency and only lipofectamine as one of tested lipofection reagents mediates a 2,5-fold increase.

In comparison to known methods and carrier systems the present invention provides a powerful transfection system which is very efficient, not time and material consuming, which is easy to perform and can be applied universally. The transfection system is not toxic for cells and/or animals and/or humans and/or plants since histones are cellular proteins. Due to the simple methode of transfection the cells are not stressed by the procedure. Since histones shuttle the nucleic acids into nuclei of eukaryotic cells the majority of the nucleic acids that should be transported is delivered to the nucleus while cytoplasmatic enzyms have no or only little opportunity to destroy the nucleic acids. With this transfection system applied in gene therapy the immune responses e.g. of the human/animal should be avoided or at least dramatically decreased in comparison to viral vectors such as adoviral vectors.
- Figure 1:: Construction of the plasmid pCV1 with AdMLP (adenovirus major late promotor) and the cDNA of the tat gene.
- Figure 2:: Plasmid pC15CAT with SV40 promotor, HIV-1 LTR (C15) and CAT gene (CAT).

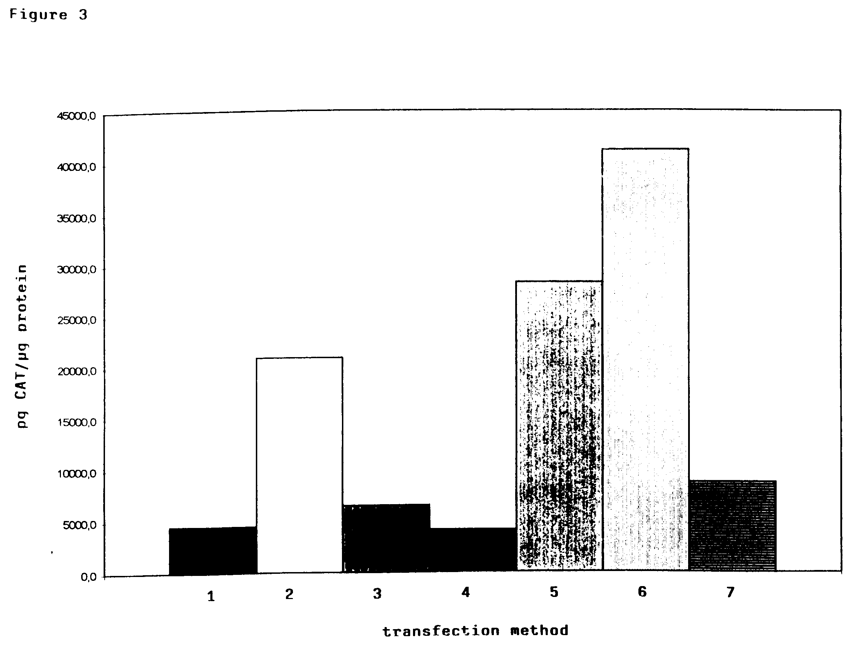

- Figure 3:: The diagram compairs the effect (measured as amount of CAT in pg/µm protein) of the best ratio of DNA : reagent of different transfection methods. The best ratio of DNA : reagent for each of the transfection method (1 to 7) was determined in separate experiments (tables 2). (1) lipofectin (20 : 35); (2) lipofectamine (20 : 35), (3) DOTAP (50 : 120), (4) Dosper (40 : 100), (5) histone 3 (40 : 175), (6) histone 4 (40 : 200) and (7) DEAE-dextran (4 : 50). The incubation time for transfection is given in example 1.

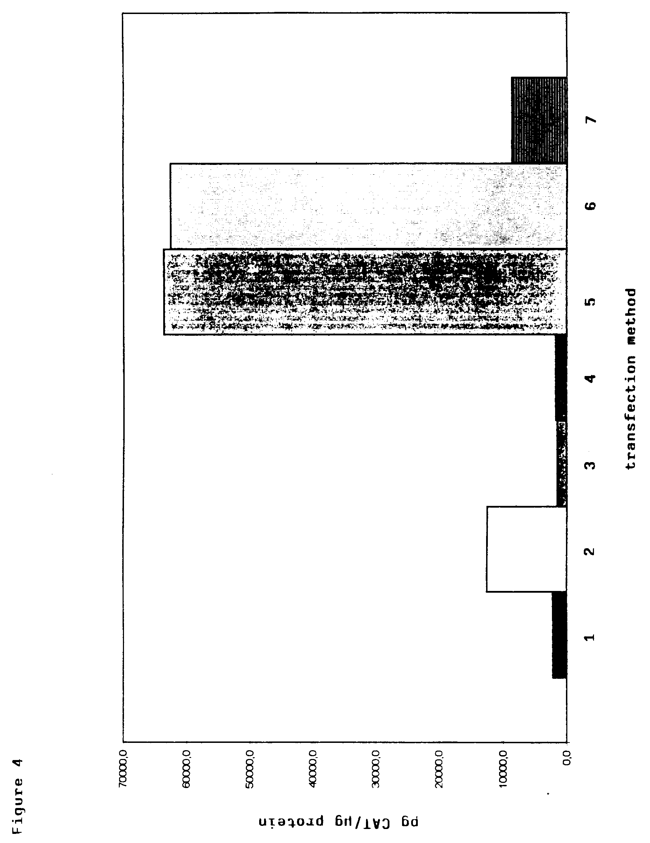

- Figure 4:: The diagram shows a compairison of the different transfection methods (1 to 7) with respect to the best incubation time for transfection. The best incubation time for each transfection methode has been determined in separate experiments (results in table 3). For these experiments in each case the best ratio of DNA : reagent has been used. (1) lipofectin (20 : 35; 5 h); (2) lipofectamine (20 : 35; 1 h), (3) DOTAP (50 : 120; 9 h), (4) Dosper (40 : 100; 8 h), (5) histone 3 (40 : 175; 4 h), (6) histone 4 (40 : 200; 8 h) and (7) DEAE-dextran (4 : 50, 1 h).

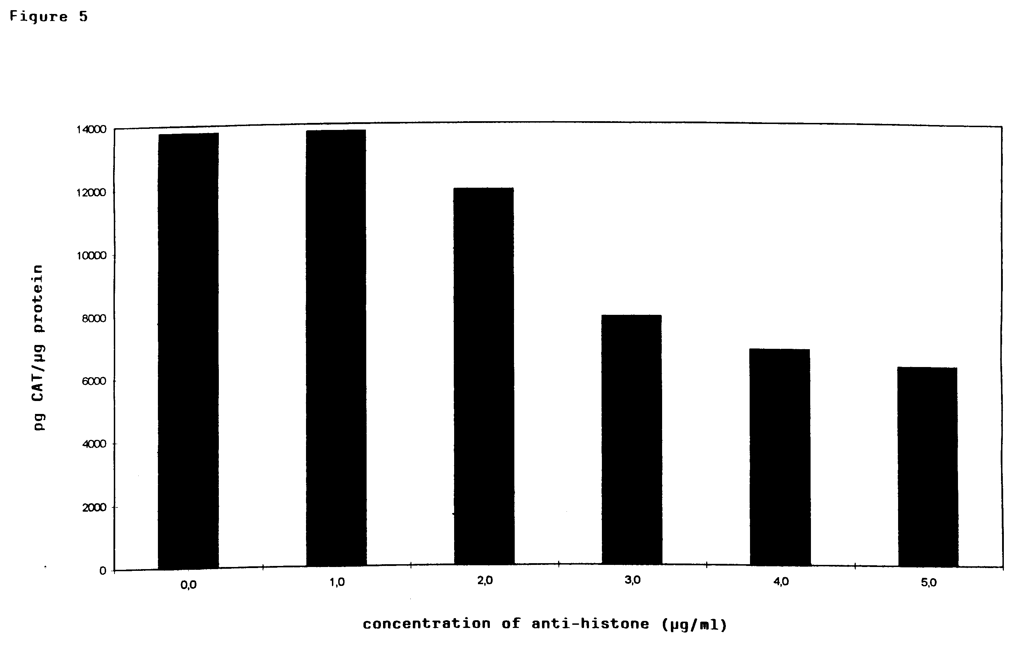

- Figure 5:: This figure shows the inhibition of transfection (histonefection with transfection system DNA/histon 3) with anti-histone antibodies (example 6).

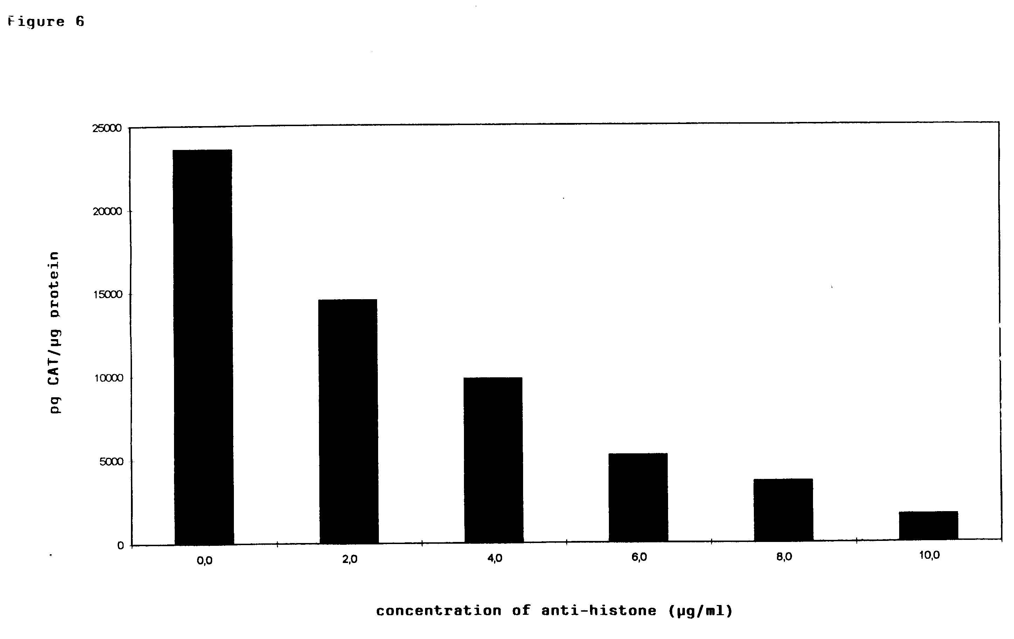

- Figure 7:: This figure shows the inhibition of transfection (histonefection with transfection system DNA/histon 4) with anti-histone antibodies (example 6).

### EXAMPLES:

### Materials:

The culture medium (RPMI 1640 with 10% fetal calf serum and 1 % penicillin and streptomycin), the wash medium (RPMI) and fetal calf serum (FCS) were supplied by GIBCO BRL, Eggenstein. Jurkat cells (clone E 6-1, Weiss *et al*.) an immortal cell line derived from T-lymphocytes were cultured in culture medium as described. ¹⁴C-acetyl-CoA (4 mCi/mmol) was obtained from NEN/Dupont (Dreieich). All other reagents were analytic-grade of high purity obtained from Boehringer Mannheim, Mannheim (DOTAP and Dosper), FLUKA, Neu-Ulm (polylysine and histones 3 and 4), GIBCO BRL (lipofectin and lipofectamine) or from Sigma, Deisenhofen (DEAE-dextran).

### Plasmids:

The tat gene (clone 1) was cloned by way of cDNA-cloning, using poly (A)selected RNA from HIV-1 (BH 10) infected cells (Arya *et al*., 1985; Chandra *et al*., 1988). The plasmid pCV1 (figure 1) was obtained by inserting viral cDNA containing the tat gene (clone 1) into the mammalian expression vector pCV which contains duplicated SV40 origins of replication (ori), adenovirus major late promotor, splice sites from adenovirus and mouse immunoglobulin genes, mouse dihydrofolate reductase cDNA, and SV40 polyadenylation signal. The HIV-1 LTR-CAT construct was obtained by inserting clone 15 DNA into pSVO-CAT at the Hind III site (Arya *et al*., 1985; Arya *et al.*, 1986) and the resulting plasmid was termed pC15CAT (figure 2).

### Example 1: Comparable transfection experiments.

In transfection experiments 5 x 10⁶ Jurkat cells (with exception of DEAE-dextran experiment) per well were cultured in a 6-well tissue culture grade plate. Varying amounts between 5 and 25 µg per well of each plasmid DNA were used. To obtain varied concentrations of transfection reagents and varied ratio of DNA and reagent in a fixed final volume of transfection solution the volume of used buffer was altered. Results of these experiments are shown in table 2.
a) By using DEAE-dextran for transfection 2 x 10⁷ Jurkat cells were used. Jurkat cells were washed with serum-free medium and incubated at 37°C for 1 h in 2 ml serum-free medium containing 50 mM Tris-HCl (pH 7.3), DEAE-dextran (Mᵣ 500.000, 250 µg/ml) and 20 µg of each plasmid DNA, pCV 1 and pC15CAT. Then the cells were washed with RPMI medium (without serum) and incubated in 6 ml serum-containing medium at 37°C for 72 hrs.
b) For transfection with DOTAP (N-[1-(2,3-dioleoyloxy)-propyl]-N,N,N-trimethylammonium methylsulfate) or the following lipofection reagents the Jurkat cells were washed twice with serum-free medium and resuspended in 4,15 ml up to 4,65 ml culture medium. Simultaneously the plasmids (5 µg of each plasmid per 1×10⁶ cells) were diluted in 250 µl HEPES (20 mM). DOTAP was solved in concentrations between 5 - 30µg/ml in 100 µl to 600 µl HEPES buffer and mixed gently with the plasmid solution.The mixture was incubated for 15 min. at room temperature. The DNA-DOTAP complex was added to the resuspended cells and incubated between 1 and 24 hrs. Then the medium was substituted with fresh RPMI medium and the cells were cultured for 72 hrs at 37°C and 5 % CO₂.
c) Dosper (1,3-dioleoyloxy-2-(6-carboxy-spermyl)-propylamind) in concentrations between 10-50 µg/ml and 4 µg per 1x10⁶ cells of each plasmids were diluted in HBS (20 mM HEPES, 150 mM NaCl) using volumina between 625 and 1000 µl, mixed and incubated for 15 min. at room temperature. After addition of the DNA-Dosper complex to the cells, which were diluted in 1,5 ml up to 1,85 ml serum-free medium, the cells were incubated for 8 hrs at 37°C. Then 0,5 ml FCS and 4,5 ml culture medium were added to the cells and the cells were incubated for 24 hrs with 5 % CO₂. Cells were centrifugated and resuspended in 5 ml fresh culture medium and then incubated for further 48 hrs under same conditions.
d) Lipofectin (a 1:1 [w/w] mixture of DOTAP (N-[1-(2,3-dioleoyloxy)-propyl]-N,N,N-trimethylammonium chloride and dioleoyl phosphotidylethanolamine (DOPE)) or lipofectamine ( a 3:1 mixture of 2,3-dioleoyloxy-N-2-spermine carboxamidoethyl-N,N-dimethyl-1-propanammoniumtriflouroacetat (DOSPA) and dioleoyl phosphotidylethanolamine) were used in concentrations between 5 and 45 µg/ml. They were diluted in 100 µl of 20 mM HEPES. 10 µg of pCV1 and pC15ACAT were diluted in 100 µl 20 mM HEPES. DNA and reagent were mixed and incubated for 15 min. at room temperature. The mixture was added to the cells, which were suspended in 0,8 ml serum-free medium. The cells were incubated for 1 hr at 37°C. Then 4 ml RPMI medium were added and the cells were incubated for 72 hrs at 37°C and 5 % CO₂.
e) Cells, which should be transfected with histones, were resuspended in 1,05 ml up to 2,3 ml RPMI medium without serum and distributed in culture plates. Histones were diluted in 0 to 1,25 ml HBS. The plasmids (20 µg of each) were diluted in 200 µl of HBS and were added to the histone solutions. After incubation of the mixture for 20 min. the solutions of DNA-histone complexes (different ratio of DNA and histone in the solutions) were added to the cells with final concentrations of histones between 0 and 100 µg/ml. The transfection solutions were incubated between 0 and 24 hrs at 37°C and 5 % CO₂. Then 275 µl of FCS and 2,5 ml RPMI medium were added and the cells were cultered for 24 hrs at 37°C and 5 % CO₂. The cells were centrifugated and resuspended in 6 ml fresh culture medium and incubated for further 48 hrs at 37°C and 5 % CO₂.
f) Transfection experiments were performed with polylysine at the same conditions as with histones.

After transfection and incubation, cells were washed with phosphate buffered saline and suspended in 130 µl of 0.25 M Tris-HCl (pH 7.8). Cellular extracts were prepared by three cycles of freezing (in liquid nitrogen) and thawing (37°C). All cellular extracts were adjusted to equal protein concentration and heated for 15 min at 65°C to inactivate deacetylases in the extract.

### Example 2: Evaluation of transfection experiments.

### a) Protein quantification

The protein quantification of each cell lysate was carried out by Bradford method (Bradford, M.M. (1976) Anal. Biochem. 72, 248-254) method, which was performed in dublicate using 10 µl of cell lysate. Absorption readings (A₅₉₅) were converted to concentrations by comparison with a bovine serum albumin (Sigma) (20-100 µg/ml) standard curve after substraction of background values.

### b) CAT assay

For CAT assays, 30 µl of the cell extract were mixed with 20 µl of 100 mM Tris-HCl (pH 7.8) in a 6 ml glass scintillation vial. The vial was transferred to a water bath set to 37°C and 200 µl of freshly prepared CAT-reaction mixture (100 µl of 0.25 M Tris-HCl, pH 7.8; 50 µl of 5 mM chloramphenicol, 10 µl ¹⁴C-acetyl-CoA, 0.1 µCi; and 40 µl water) were added. To this mixture, 5 ml of a water-immiscible scintillation fluid (Econofluor, NEN/Dupont) was carefully overlaid and incubated at 37°C for 4 h. All transfections were performed minimum in dublicate for each set of experiments.

### Example 3: Determination of optimal histone to DNA ratio and optimal transfection rate by gel shift assay (gel retardation assay)

The optimal mass ratio of histone to DNA (histone:DNA) was detected in a gel retardation assay. The samples with a ratio from 1:1 up to 5:1 were electrophoresed on a 1 % agarose gel. In a gel retardation assay the migration of plasmid DNA is increasingly retarded as concentrations of the histones increased.

0,5 µg of each plasmid were mixed with 1 µg up to 5 µg of various histones in 20 µl of HBS, incubated for 20 min. at room temperature. After adding of 10 µl loading buffer (50 % 1xTE, 50 % glycerol and 0,05 % of brome phenolblue and xylene xyanol) and mixing the samples were electrophoresed on an 1 % agarose gele. The electrophoresed gele was stained with ethidium bromide and visualized with UV (l = 254 nm). The visualized gel was scanned with a video camera by use of the Imager from Appligene (France) and was saved as tiff-file or printed by a thermo printer.

The optimal histone:DNA ratio that resulted in complete protein-DNA binding was the same for both tested histones. With both histones DNA was completly retarded at a ratio of 5:1.

### Example 4: Determination of transfection rate using varying reagent to DNA ratios

Jurkat cells were transfected with histone 3/plasmids pCV1 and pC15ACAT (1:1), histone 4/plasmids (CpCV1 + pC15ACAT, 1:1) and polylysine/plasmids at various protein:DNA ratios. For both histones, the optimal (histone 4) or nearly the optimal (histone 3) protein-DNA binding ratio also gave the best reporter gene expression in Jurkat cells. Experiments with polylysine leaded to no transfection in Jurkat cells.

According to standard protocols obtained from the companies, the optimal reagent to DNA ratio was used to determine the best transfection efficiency for each condition. In these experiments a fixed amount of plasmid DNA was used. Comparison of the results (table 2; table 1) demonstrates that lipofectamine gave 2,5 fold better transfection rate compared to DEAE-dextran. All other reagents leaded to a significant lower transfection efficiency. DEAE-dextrane which used as standard leds to 8718 pg CAT/µg protein (table 1).

### Example 5: Determination of the optimal incubation time

By using optimal reagent: DNA ratios for all transfection reagents the transfection efficiency at various incubation times was determined according to the procedure described in example 1 and 2. The results (table 3; table 1) indicated that lipofectamine and both histones leaded to better transfection efficiencies than DEAE-dextrane. More, the results show that histones are twice or more times better than lipofectamin and nearly 7-fold better than transfection with DEAE-dextrane. Transfection of Jurkat cells by using histones as carrier system leaded to the best transfection efficiency.Transfection experiments with a mixture of both histones under same conditions like alone not resulted in a better transfection rate.

### Example 6: Inhibition of transfection (histonefection) by monoclonal anti-histone antibodies.

Inhibition of transfection by anti-histone antibodies prooves that histones are responsible for the transfer of nucleic acids. Histones H3 and/or H4 in HBS were incubated with anti-histone antibodies (mouse-IgG1 against the panepitope, available from Boehringer Mannheim, No. 1 471, 519, Biochemicals Catalog 1995) (concentration of anti-histone antibodies 0-5µg/ml) for 1 hour at room temperature. The rest of the procedure was performed as described in example 1 (incubation with plasmids, transfection).

### Example 7: Preparation of histones from animal tissue.

Tissue from calf thymus is homogenized in 0.9 % NaCl-solution and centrifuged at 1100 g. The pellet is washed five times with 0.9 % NaCl. The pellet is homogenized with 5 % perchloric acid and zentrifuged at 1100 g two times. The pellet is suspended in ethanol and incubated at 4°C for 18 hours. The suspension is centrifuged (1100 g) and the pellet is washed with ethanol twice. The supernatants from ethanol extractions are collected, pooled and centrifuged at 2500 x g. The supernatant is mixed with aceton (5 x volume) and then with concentrated HCl. The pellet is washed with aceton and dissolved in H₂O. Then ethanol (endconcentration 90%) and HCl (end concentration 0.25 N) are added and the solution is dialysed 18 hours against ethanol. Then the solution is again dialysed under the same conditions for 4 hours. The pellet (f3) is washed once with ethanol and three times with aceton and dried in vakuum. The supernatant is mixed with aceton (3 x volume) and the pellet (f2a) is washed twice with aceton and dried in vakuum.

**Table 1**

| | | | | |
|---|---|---|---|---|
| In this table the optimized conditions and transfection efficiency of all transfection methods received from the mesure of CAT expression described above are summarized. | | | | |

| transfection reagent | ratio of DNA to transfection reagent | time of incubation [h] | amount of each plasmid [µg/10⁶ cells] | pg CAT/µg protein |
|---|---|---|---|---|
| DEAE-dextrane (Sigma) | 4:50 | 1 | 1 | 8717,9 |
| lipofektin (GIBCO BRL) | 20:35 | 1 | 1 | 2161,1 |
| lipofectamine (GIBCO BRL) | 20:35 | 1 | 1 | 12499,7 |
| DOTAP (Boehringer Mannheim) | 50:120 | 9 | 5 | 1500,4 |
| Dosper (Boehringer Mannheim) | 40:100 | 8 | 4 | 1715,2 |
| histone 3 Johns Fraction III (Fluka) | 40:175 | 4 | 4 | 63688,5 |
| histone 4 (Fluka) | 40:200 | 8 | 4 | 62555,2 |
| poly-Lys (Fluka) | 40:0 - 40:250 | 6 | 4 | 0 |

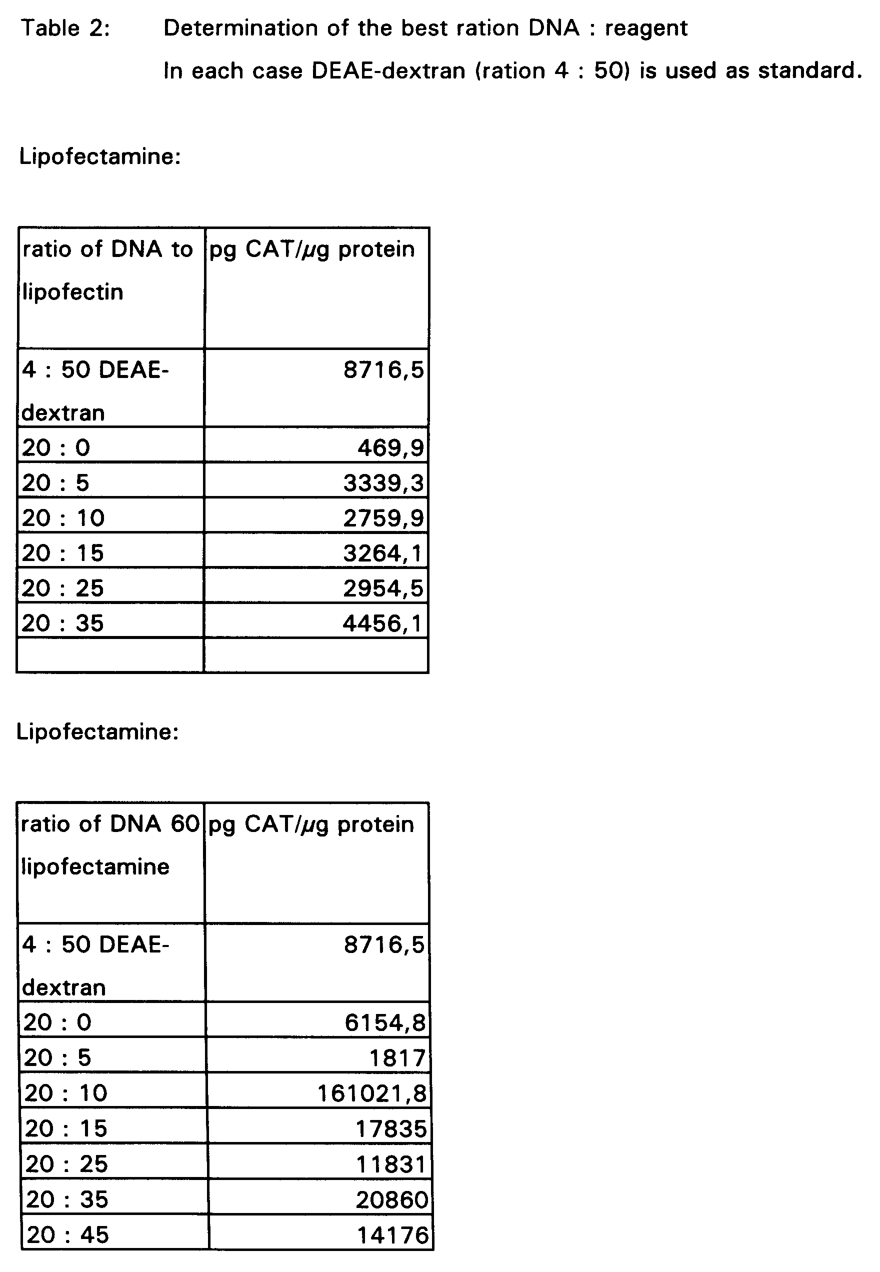

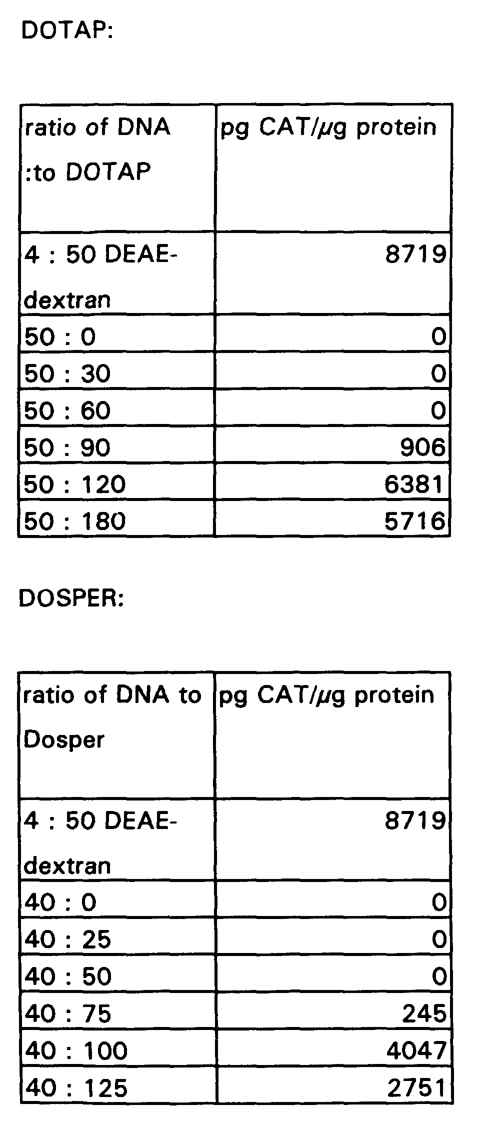

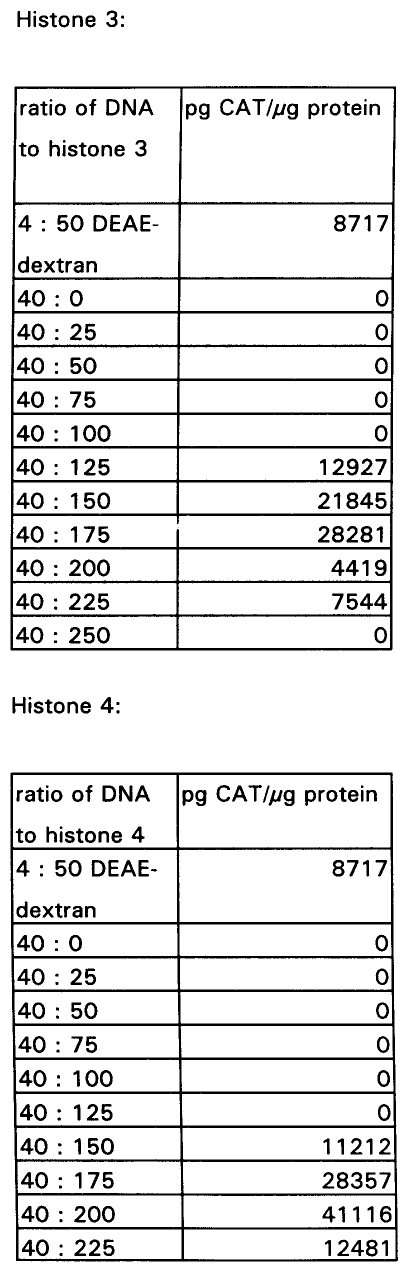

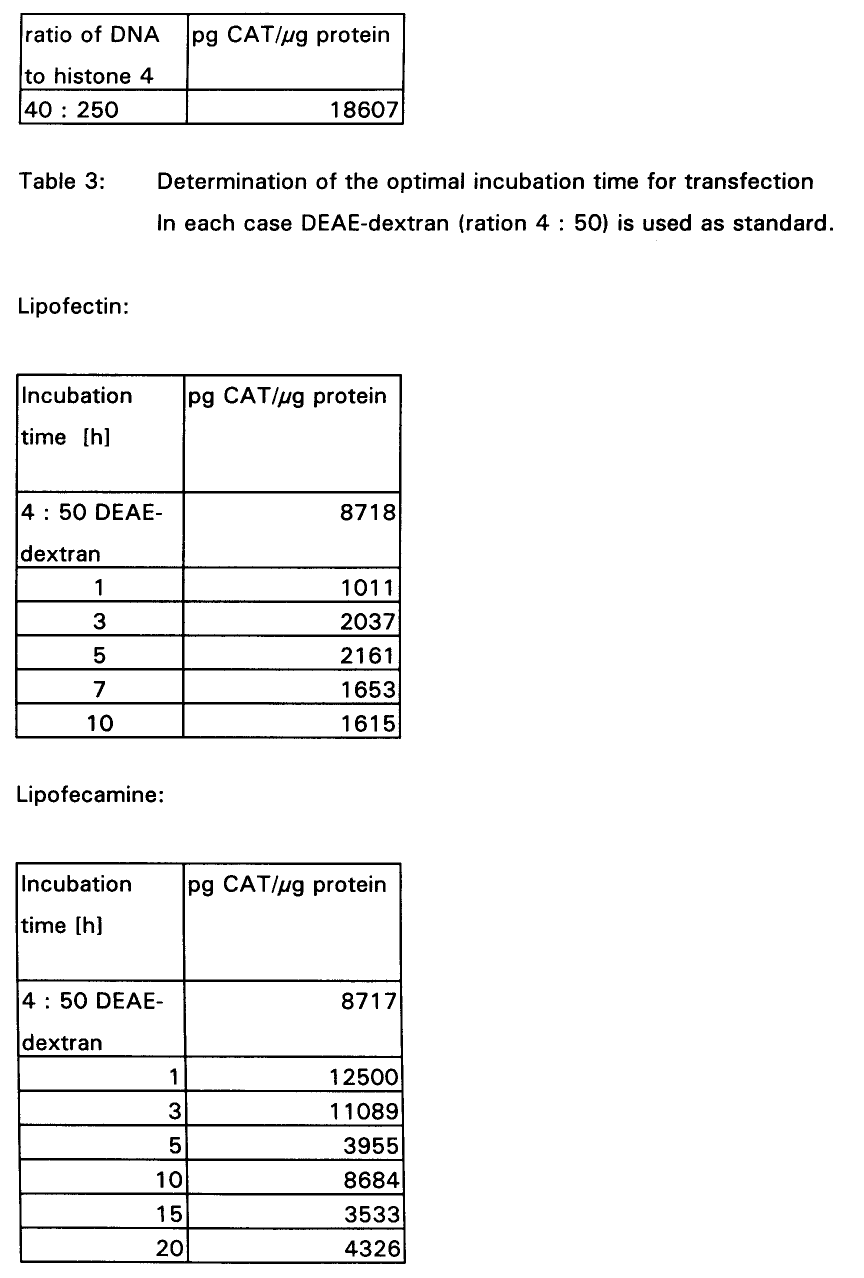

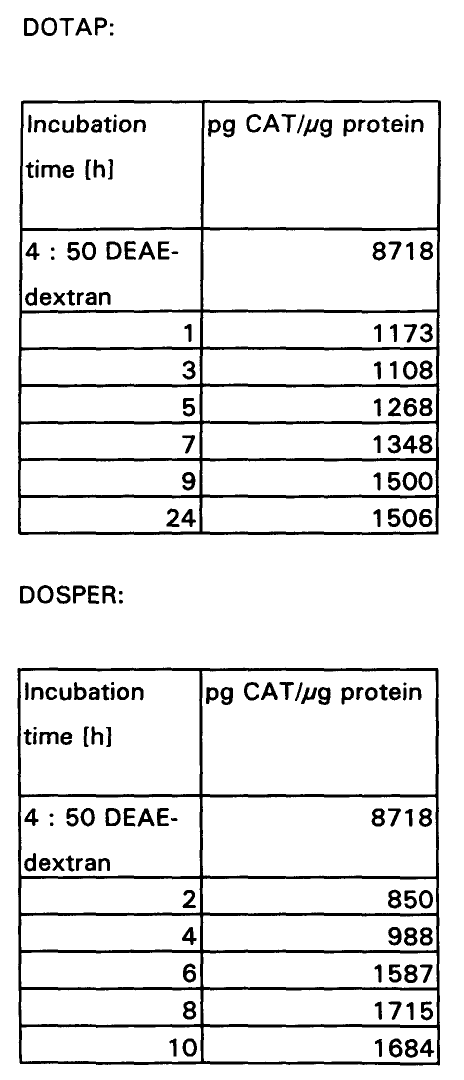

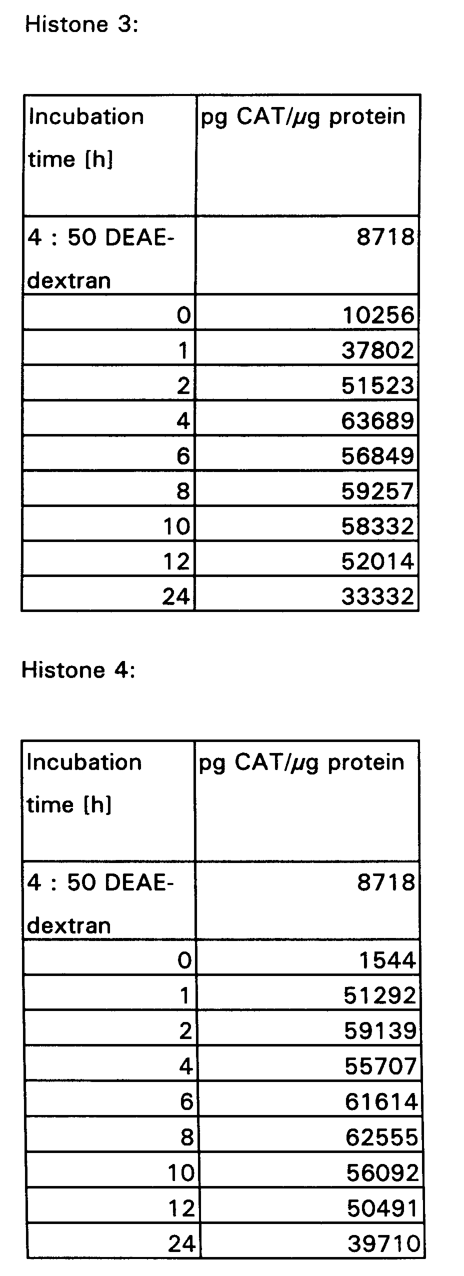

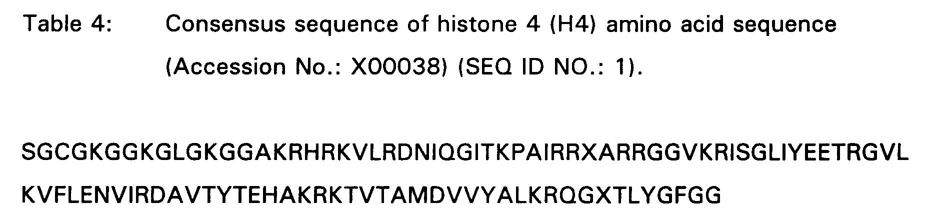

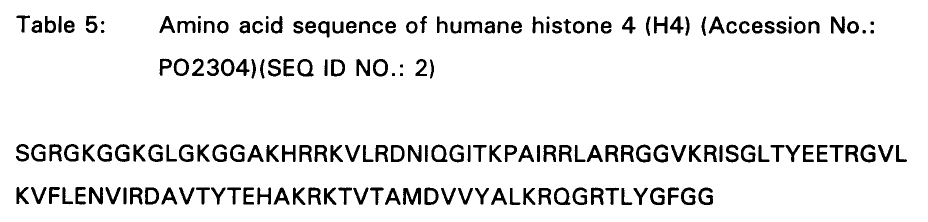

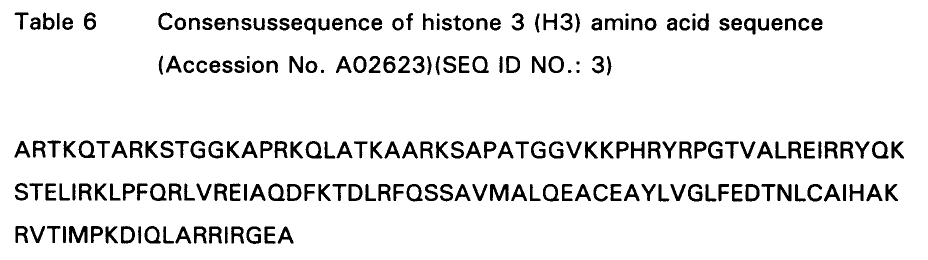

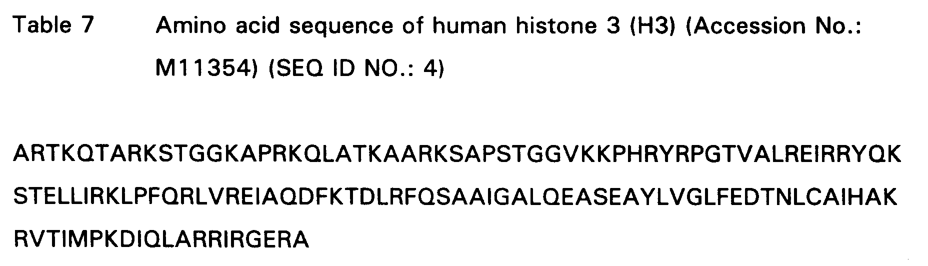

### References:

Arya, S. K. , Guo, C. , Josephs, S. F. , Wong-Staal, F. Trans-activator gene of human T-lymphotropic virus type III (HTLV III). Science 229, 69-73 (1985)
Arya, S. K. and Gallo, R. C. Three novel genes of human T-lymphotropic virus type III: immune reactivity of their products with sera from acquired immune deficiency syndrome patients. Proc. Natl. Acad. Sci. U. S. A. 83, 2209-2213 (1986)
Bonner, W. M. Protein migration into nuclei. II. Frog oocyte nuclei accumulate a class of microinjected oocyte nuclear proteins and exclude a class of microinjected oocyte cytoplasmic proteins. J. Cell Biol. 64, 431-437 (1975)
Chandra A. , Demirhan, I. , Arya, S. K. , and Chandra P. D-Penicillamine inhibits transactivation of human immunodeficiency virus type-1 (HIV-1) LTR by transactivator protein. FEBS Letters 236, 282-286 (1988)
Chen, C. A. and Okayama, H. Calcium phosphate-mediated gene transfer: a highly efficient transfection system for stably transforming cells with plasmid DNA. BioTechniques 6, 632-638 (1988)
Chen, J. , Stickles, R.J. , and Daichend, K. A. Galactosylated histone-mediated gene transfer and expression. Human Gene Therapy 5, 429-435 (1994) Choi, O.-R. B. and Engel, J. D. Developmental regulation of beta-globin gene switching. Cell 55, 17-26 (1988)
Cotten, M. , Langle-Rouault, F. , Kirlappos, H. , Wagner, E. , Mechtler, K. , Zenke, M. , Beug, H. , and Birnstiel, M. L. Transferrin-polycation-mediated introduction of DNA into human leukemic cells: Stimulation by agents that affect the survival of transfected DNA or modulate transferrin receptor levels. Proc. Natl. Acad. Sci. U. S. A. 87, 4033-4037 (1990)
Cotten, M. , Wagner, E. , Zatloukal, K. , and Birnstiel, M. L. Chicken adenovirus (CELO virus) particles augment receptor-mediated DNA delivery to mammlian cells and yield exceptional levels of stable transformants. J. Virolgy 67, 3777-3785 (1993)
Cotten, M. , Wagner, E. , Zatloukal, K. , Phillips, S. , Curiel, D. T. , and Birnstiel, M. L. High-efficiency receptor-mediated delivery of small and large (48 kilobase) gene constructs using the endosome-disruption activity of defective or chemically inactivated adenovirus particles. Proc. Natl. Acad. Sci. U. S. A. 89, 6094-6098 (1992)
Curiel, D. T. , Agrawal, S. , Romer, M. U. , Wagner, E. , Cotten, M. , Birnstiel, M. L. , and Boucher, R. C. Gene transfer to respiratory epithelial cells via the receptor-mediated endocytosis pathway. Amer. J. Resp. Cell & Mol. Biol. 6, 247-252 (1992)
Curiel, D. T. , Agrawal, S. , Wagner, E. , and Cotten, M. Adenovirus enhancement of transferrin-polylysine-mediated gene delivery. Proc. Natl. Acad. Sci. U. S. A. 88, 8850-8854 (1991)
Demirhan, I. , Hasselmayer, O. , Hofmann, D. , Chandra, A. , Svinarchuk, F. P. , Vlassov, V. V. , Engels, J. , and Chandra P. Gene-targeted inhibition of transactivation of human immunodeficiency virus type-1 (HIV-1) LTR by antisense oliginucleotides. Virus Genes 9, 113-119 (1994)
Dingwall, C. and Allan, J. Accumulation of the isolated carboxyterminal domain of histone 1 H1 in the Xenopus oocyte nucleus. EMBO J. 3, 1933-1937 (1984) Felgner,P. L. , Gadek, T. R. , Holm, M. , Roman, R. , Chan, H. W. , Wenz, M. , Northrop, J. P. , Ringold, G. M. , and Danielsen, M. Lipofection: A highly efficient, lipid-mediated DNA-transfection procedure. Proc. Natl. Acad. Sci. U. S. A. 84, 7413-7417 (1987)
Fraley, R. and Papahadjopoulus, D. Liposomes: the development of a new carrier sytem for introducing nucleic acid into plant and animal cells. Curr. Top. Microbiol.Immunol. 96, 171-191 (1982)
Graessmann, M. and Graessmann A. (1986). "Microinjection and Organelle Transplantation Techniques" (Celis, J. E. , Graessmann, A. , and Loyter, A. , eds.) pp. 3-13. Academia, London, 1986
Graham, F. L. and van der Eb, A. J. A new technique for the assay of infectivity of human adenovirus 5 DNA. Virology 52, 456-467 (1973)
Holt, C. E. , Garlick, N. , and Cornel, E. Lipofection of cDNAs in the embryonic vertebrate central nervous system. Neuron 4, 203-214 (1990)
Kaneda, Y. , Iwai, K. , and Uchida, T. Increased expression of DNA cointroduced with nuclear protein in adult rat liver. Science 243, 375-378 (1989)
Kawai, S. and Nishizawa, M. New procedure for DNA transfection with polycation and dimethyl sulfoxide. Mol. Cell Biol. 4, 1172-1174 (1984)
Keown, W. A. , Campbell, C. R. , and Kucherlapati, R. S. Methods of introducing DNA into mammalien cells. Methods Enzymol. 185, 527-537 (1990)
Loyter, A. , Scangos, G. A. , and Ruddle, F. H. Mechanisms of DNA uptake by mammalian cells: fate of exogenously added DNA monitored by the use of fluorescent dyes. Proc. Natl. Acad. Sci. U. S. A. 79, 422-426 (1982)
Machy, P. , Lewis, F. , McMillan, L. , and Jonak, Z. L. Gene transfer from targeted liposomes to specific lymphoid cells by electroporation. Proc. Natl. Acad. Sci. U. S. A. 85, 8027-8031 (1988)
Mannino, R. , J. and Gould-Fogerite, S. Liposome mediated gene transfer. BioTechnique 6, 682-690 (1988)
Marceau, F. , Boisjoly, H. M. , Wagner, E. , Lille, S. , and Roy, R. Long-term culture and characterization of human limbal microvascular endothelial cells. Exper. Eye Res. 51, 645-650 (1990)
McCutchan, J. and Pagano, J. S. Enchancement of the infectivity of simian virus 40 deoxyribonucleic acid with dimethylaminoethyl-dextran. J. Natl. Cancer Inst. 41, 351-357 (1968)
Moreland, R. B. , Langevin, G. I. , Singer, R. H. , Garcea, R. L. , and Hereford, L. M. Amino acid sequences that determine the nuclear localization of yeast histone 2B. Mol. Cell. Biol. 7, 4048-4057 (1987)
Neumann, E. , Schaefer-Ridder, M. , Wang, Y. , and Hofschneider, P. H. Gene transfer into mouse lyoma cells by electroporation in high electric fields. EMBO J. 1, 841-845 (1982)
Nicolau, C. , Legrand, A. T. , and Grosse, E. Liposomes as carriers for in vivo gene transfer and expression. Methods Enzymol. 152, 157-176 (1987)
Othani, K. , Nakamura, M. , Saito, S. , Nagata, K. , Sugamura, T. , and Hinuma, Y. Electroporation: application to human lymphoid cell lines for stable introduction of a transactivator gene of human T-cell leukemia virus type I. Nucleic Acids Res. 17, 1589-1604 (1989)
Plank, C. , Oberhausen, B. , Mechtler, K. , Koch, C. , and Wagner, E. The influence of endosome-disruptive peptides on gene transfer using synthetic virus-like gene transfer systems. J. Biol. Chem. 269, 12918-12924 (1994)
Wagner, E. , Cotten, M. , Foisner, R. , and Birnstiel, M. L. Transferrin-polycation-DNA complexes: The effect of polycations on the structure of the complexes and DNA delivery to cells. Proc. Natl. Acad. Sci. U. S. A. 88, 4255-4259 (1991)
Wagner, E. , Plank, C. , Zatloukal, K. , Cotten, M. , and Birnstiel, M. L. Influenza virus hemagglutinin HA-2 N-terminal fusogenic peptides augment gene transfer by transferrin-polylysine-DNA complexes: Toward a synthetic virus-like gene-transfer vehicle. Proc. Natl. Acad. Sci. U. S. A. 89, 7934-7938 (1992a)
Wagner, E. , Zatloukal, K. , Cotten, m. , Kirlappos, H. , Mechtler, K. , Curiel, D. T., and Birnstiel, M. L. Coupling of adenovirus to transferrin-polylysine/DNA complexes greatly enhances receptor-mediated gene delivery and expression of transfected genes. Proc. Natl. Acad. Sci. U. S. A. 89, 6099-6103 (1992b)
Wagner, E. , Zenke, M. , Cotten, M. , Beug, H. , and Birnstiel, M. L. Transferrin-polycation conjugates as carriers for DNA uptake into cells. Proc. Natl. Acad. Sci. U. S. A. 87, 3410-3414 (1990)
Wang, C.-Y. and Huang, L. pH-sensitive immunoliposomes mediate target-cell-specific delivery and controlled expression of a foreign gene in mouse. Proc. Natl. Acad. Sci. U. S. A. 84, 7851-7855 (1987)
Weiss, R. A. , Teich, N. M. , Varmus, A. E. and Coffin, J. RNA Tumor Virus Molecular Biology of Tumor Virus: RNA Tumor Virus, Cold Spring Harbor Lab., Vol. 1 and 2, New York, 1985
Wu, C. H. , Wilson, J. M. , and Wu, G. Y. Targeting genes: delivery and persistent expression of foreign gene driven by mammalian regulatory elements in vivo. J. Biol. Chem. 264, 16985-16987 (1989)
Wu, G. Y. , Wilson, J. M. , Shalaby, F. , Grossman, M. , Shafritz, D. A. , and
Wu, C. H. Receptor-mediated gene delivery in vivo. Partial correction of genetic analbuminemia in Nagase rats. J. Biol. Chem. 266, 14338-14342 (1991)
Wu, G. Y. , Zhan, P. , Sze, L. L. , Rosenberg, A. R. , and Wu, C. H. Incorporation of adenovirus into ligand-based DNA carrier system results in retention of original receptor specificity and enhances targeted gene expression. J. Biol. Chem. 269, 11542-11546 (1994)
Zauner, W. , Blaas, D. , Kuechler, E. , and Wagner, E. Rhinovirus-mediated endosomal release of transfection complexes. J. Virology 69, 1085-1092 (1995)
Zenke, M. , Steinlein, P. , Wagner, E. , Cotten, M. , Being, H. , and Birnstiel, M. L. Receptor-mediated endocytosis of transferrin-polycation conjugates: An efficient way to introduce DNA into hematopoietic cells. Proc. Natl. Acad. Sci. U. S. A. 87, 3655-3659 (1990)

## Claims

1. A transfection system that comprises a histone protein or a derivate thereof and a nucleic acid.

2. A transfection system as claimed in claim 1, wherein the histone is histone 3 (H3) or a derivate thereof.

3. A transfection system as claimed in claim 1, wherein the histone is histone 4 (H4) or a derivate thereof.

4. A transfection system as claimed in one or more of claims 1 to 3, wherein the histone is acetylated.

5. A transfection system as claimed in one or more of claims 1 to 4, wherein the histone has been isolated from tissue.

6. A transfection system as claimed in claim 5, wherein the histone has been isolated from calf thymus.

7. A transfection system as claimed in one or more of claims 1 to 5, wherein the histone has been produced by recombinant expression of histone-encoding DNA in prokaryotic or eukaryotic cells.

8. A transfection system as claimed in claim 7, wherein the histone has been produced by recombinant expression of human histone-cDNA in prokaryotic or eukaryotic cells.

9. A transfection system as claimed in one or more of claims 1 to 8, wherein the nucleic acid comprises a DNA.

10. A transfection system as claimed in claim 9, wherein the DNA comprises sequences that encode for one or more proteins.

11. A transfection system as claimed in claim 10, wherein at least one of the encoded proteins is an enzym.

12. A transfection system as claimed in claim 11, wherein the encoded protein is Chloramphenicol-Acetyl-Transferase (CAT).

13. A transfection system as claimed in one or more of claims 10 to 12, wherein at least one of the encoded proteins is a trans-activating factor.

14. A transfection system as claimed in claim 13, wherein the transactivating factor is the HIV-1 protein tat.

15. A transfection system as claimed in one or more of claims 9 to 14, wherein the DNA comprises one or more promotors for the controlled expression of encoded proteins.

16. A transfection system as claimed in claim 15, wherein at least one promotor is a constitutive promotor.

17. A transfection system as claimed in one or more of claims 15 and 16, wherein at least one promotor is an inducible promotor.

18. A transfection system as claimed in claim 17, wherein the inducible promotor is turned on by a specific trans-activating factor.

19. A transfection system as claimed in one or more of claims 17 and 18, wherein the inducible promoter is the HIV-1 long terminal repeat (LTR).

20. A transfection system as claimed in one or more of claims 9 to 19, wherein the nucleic acid comprises plasmids.

21. A transfection system as claimed in claim 20 comprising the plasmids pCV1 and pC15CAT.

22. A transfection system as claimed in one or more of claims 1 to 21, wherein the nucleic acid comprises a RNA.

23. A transfection system as claimed in one or more of claims 1 to 22, wherein the nucleic acid comprises an oligonucleotides.

24. A method of making a transfection system according to one or more of claims 1 to 23 having the ability to transfer nucleic acids into a cell.

25. A method of making a transfection system according to claim 24 by mixing histones with nucleic acids.

26. A method of making a recombinant cells by using a transfection system according to one or more of claims 1 to 23.

27. Use of histones to transfer nucleic acids into cells.

28. Use of a transfection system as claimed in one or more of claims 1 to 23 for transferring nucleic acid into a cell.

29. Use of a transfection system as claimed in claim 24 wherein the cell is an eukaryotic cell.

30. Use of a transfection system as claimed in one or more of claims 1 to 23 for the preparation of a pharmaceutical composition that can be used for DNA vaccination.

31. Use of a transfection system as claimed in one or more of claims 1 to 23 in gene therapy.

32. Use of transfection system as claimed in one or more of claims 1 to 23 for the preparation of a pharmaceutical composition that can be used in gene therapy.


SEQUENCE LISTING

(1) GENERAL INFORMATION:

(i) APPLICANT:
(A) NAME: Hoechst Marion Roussel Deutschland GmbH
(B) STREET: -
(C) CITY: Frankfurt
(D) STATE: -
(E) COUNTRY: Germany
(F) POSTAL CODE (ZIP): 65926
(G) TELEPHONE: 069-305-7072
(H) TELEFAX: 069-35-7175
(I) TELEX: -

(ii) TITLE OF INVENTION: New transfection system for the transfer of
nucleic acids into encaryotic cells

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..102

(xi) SEQUENCE DESCRIPTION: SEQ ID NO: 1:

Ser Gly Cys Gly Lys Gly Gly Lys Gly Leu Gly Lys Gly Gly Ala Lys
1               5                   10                  15

Arg His Arg Lys Val Leu Arg Asp Asn Ile Gln Gly Ile Thr Lys Pro
            20                  25                  30

Ala Ile Arg Arg Xaa Ala Arg Arg Gly Gly Val Lys Arg Ile Ser Gly
        35                  40                  45

Leu Ile Tyr Glu Glu Thr Arg Gly Val Leu Lys Val Phe Leu Glu Asn
    50                  55                  60

Val Ile Arg Asp Ala Val Thr Tyr Thr Glu His Ala Lys Arg Lys Thr
65                  70                  75                  80

Val Thr Ala Met Asp Val Val Tyr Ala Leu Lys Arg Gln Gly Xaa Thr
                85                  90                  95

Leu Tyr Gly Phe Gly Gly
            100

(2) INFORMATION FOR SEQ ID NO: 2:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..102

(xi) SEQUENCE DESCRIPTION: SEQ ID NO: 2:

Ser Gly Arg Gly Lys Gly Gly Lys Gly Leu Gly Lys Gly Gly Ala Lys
1               5                   10                  15

His Arg Arg Lys Val Leu Arg Asp Asn Ile Gln Gly Ile Thr Lys Pro
            20                  25                  30

Ala Ile Arg Arg Leu Ala Arg Arg Gly Gly Val Lys Arg Ile Ser Gly
        35                  40                  45

Leu Thr Tyr Glu Glu Thr Arg Gly Val Leu Lys Val Phe Leu Glu Asn
    50                  55                  60

Val Ile Arg Asp Ala Val Thr Tyr Thr Glu His Ala Lys Arg Lys Thr
65                  70                  75                  80

Val Thr Ala Met Asp Val Val Tyr Ala Leu Lys Arg Gln Gly Arg Thr
                85                  90                  95

Leu Tyr Gly Phe Gly Gly
            100

(2) INFORMATION FOR SEQ ID NO: 3:

(i) SEQUENCE CHARACTERISTICS:
(A) LENGTH: 134 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..134

(xi) SEQUENCE DESCRIPTION: SEQ ID NO: 3:

Leu Ala Arg Thr Lys Gln Thr Ala Arg Lys Ser Thr Gly Gly Lys Ala
1               5                   10                  15

Pro Arg Lys Gln Leu Ala Thr Lys Ala Ala Arg Lys Ser Ala Pro Ala
            20                  25                  30

Thr Gly Gly Val Lys Lys Pro His Arg Tyr Arg Pro Gly Thr Val Ala
        35                  40                  45

Leu Arg Glu Ile Arg Arg Tyr Gln Lys Ser Thr Glu Leu Ile Arg Lys
    50                  55                  60

Leu Pro Phe Gln Arg Leu Val Arg Glu Ile Ala Gln Asp Phe Lys Thr
65                  70                  75                  80

Asp Leu Arg Phe Gln Ser Ser Ala Val Met Ala Leu Gln Glu Ala Cys
                85                  90                  95

Glu Ala Tyr Leu Val Gly Leu Phe Glu Asp Thr Asn Leu Cys Ala Ile
            100                 105                 110

His Ala Lys Arg Val Thr Ile Met Pro Lys Asp Ile Gln Leu Ala Arg
        115                 120                 125

Arg Ile Arg Gly Glu Ala
    130

(2) INFORMATION FOR SEQ ID NO: 4:

(i) SEQUENCE CHARACTERISTICS:
(A) LENGTH: 135 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..135

(xi) SEQUENCE DESCRIPTION: SEQ ID NO: 4:

Ala Arg Thr Lys Gln Thr Ala Arg Lys Ser Thr Gly Gly Lys Ala Pro
1               5                   10                  15

Arg Lys Gln Leu Ala Thr Lys Ala Ala Arg Lys Ser Ala Pro Ser Thr
            20                  25                  30

Gly Gly Val Lys Lys Pro His Arg Tyr Arg Pro Gly Thr Val Ala Leu
        35                  40                  45

Arg Glu Ile Arg Arg Tyr Gln Lys Ser Thr Glu Leu Leu Ile Arg Lys
    50                  55                  60

Leu Pro Phe Gln Arg Leu Val Arg Glu Ile Ala Gln Asp Phe Lys Thr
65                  70                  75                  80

Asp Leu Arg Phe Gln Ser Ala Ala Ile Gly Ala Leu Gln Glu Ala Ser

                85                  90                  95
Glu Ala Tyr Leu Val Gly Leu Phe Glu Asp Thr Asn Leu Cys Ala Ile
            100                 105                 110
His Ala Lys Arg Val Thr Ile Met Pro Lys Asp Ile Gln Leu Ala Arg
        115                 120                 125
Arg Ile Arg Gly Glu Arg Ala
    130                 135